# EUROPEAN PATENT APPLICATION

(11) **EP 4 675 249 A1**
(43) Date of publication of application: **07.01.2026**
(21) Application number: 24763453.8
(22) Date of filing: 26.01.2024
(51) Int. Cl.: G01N 1/10, B04B 5/02, B04B 13/00, C12M 1/00, C12M 1/26, G01N 37/00

(54) **BIOLOGICAL SAMPLE SEPARATION CONTAINER, BIOLOGICAL SAMPLE SEPARATION CONTROL DEVICE, BIOLOGICAL SAMPLE SEPARATION CONTROL METHOD, AND BIOLOGICAL SAMPLE SEPARATION CONTROL PROGRAM**

(30) Priority: 27.02.2023 JP 2023028730
(71) Applicant: PHC HOLDINGS CORPORATION, Tokyo 100-8403 (JP)
(72) Inventor: JOHNO, Masahiro, Toon-shi, Ehime 791-0395 (JP)
(74) Representative: Novagraaf International SA
(86) International application number: PCT/JP2024/002452
(87) International publication number: WO 2024/180968

(57) **Abstract**

A container (30) is provided with an isolation chamber (33), a collection chamber (34), a waste liquid chamber (35), a siphon flow path (33a), and a siphon flow path (34a). The isolation chamber (33) holds a specific gravity adjusting agent (L2) and blood (L1), which have been layered. The collection chamber (34) holds a centrifuged mononuclear cell layer (L4), some of the specific gravity adjusting agent (L2), and plasma (L5), and allows the mononuclear cell layer (L4) to settle in a liquid after being suspended. The waste liquid chamber (35) holds the supernatant liquid of the suspension as waste liquid. The siphon flow path (33a) connects the isolation chamber (33) and the collection chamber (34), and uses siphoning to send a liquid containing the mononuclear cell layer (L4). The siphon flow path (34a) connects the collection chamber (34) and the waste liquid chamber (35), and uses siphoning to send the waste liquid.

## Description

### TECHNICAL FIELD

The present invention relates to a biological sample isolation container and a biological sample isolation control device, which are used in a state of having been placed in a rotating device and under centrifugal force.

### BACKGROUND ART

Conventionally, to isolate a specific component contained in blood (biological sample), the blood or the like is placed in a container such as a centrifuge tube, and centrifugal isolation is performed.

The task of removing and collecting a specific component of a biological sample that has been isolated into components by centrifugation from a container is sometimes performed manually by an operator.

For instance, Patent Literature 1 discloses a component isolation device and a component isolation method for isolating a specific component by centrifugal isolation of blood using a specific gravity adjusting agent, in which red blood cells, white blood cells, and plasma components in the blood are isolated, and the plasma portion is isolated using channel flow paths and valves for the various components, after which the specific component, white blood cells, is isolated out.

### CITATION LIST

### PATENT LITERATURE

Patent Literature 1: JP-A 2008-145420

### SUMMARY

However, the following problem is encountered with the above-mentioned conventional component isolation device.

With the component isolation device disclosed in the above publication, after centrifugation, the valves are opened so that the plasma, the white blood cells, and the specific gravity adjusting agent are each guided to isolate chambers.

Therefore, this was not a configuration in which only white blood cells were isolated, making it unsuitable for cancer diagnosis that uses only white blood cells. Furthermore, the white blood cells were present along with a large quantity of the specific gravity adjuster, which damaged the cells, and this was a problem because it reduced the bioavailability of the cells.

It is an object of the present invention to provide a biological sample isolation container, a biological sample isolation control device, a biological sample isolation control method, and a biological sample isolation control program with which cells can be properly isolated and collected without reducing the bioavailability of the cells.

### SOLUTION TO PROBLEM

The biological sample isolation container according to the first invention is biological sample isolation container that is placed in a rotating device and rotates around a specific rotation center to apply centrifugal force, thereby collecting a specific component contained in a biological sample that has been isolated for each component, the biological sample isolation container comprising an isolation chamber, a collection chamber, a waste liquid chamber, a first siphon flow path, and a second siphon flow path. The isolation chamber holds a reagent and a biological sample layered on the reagent. The collection chamber holds the specific component centrifuged in the isolation chamber, some of the reagent, and a first component contained in the biological sample, which have been sent from the isolation chamber, and after these are suspended, causes only the specific component to settle outward in the radial direction in which centrifugal force is applied in the liquid containing the specific component, some of the reagent, and the first component contained in the biological sample. The waste liquid chamber is disposed on the outside in the radial direction of the collection chamber around the rotation center, and holds as waste liquid a supernatant liquid of the liquid sent from the collection chamber, in a state in which only the specific component has settled in the collection chamber. The first siphon flow path connects the isolation chamber and the collection chamber, and uses siphoning to send the liquid, which contains a specific component that has been isolated for each component by centrifuging of the biological sample and reagent, from the isolation chamber to the collection chamber. The second siphon flow path connects the collection chamber and the waste liquid chamber, and uses siphoning to send the waste liquid from the collection chamber to the waste liquid chamber, out of the liquid stored in the collection chamber.

The biological sample isolation control device according to the second invention is the biological sample isolation control device that controls the rotation of a rotating device in which a biological sample isolation container is placed according to the first invention, comprising a rotation control unit that controls the drive unit of the rotating device.

The biological sample isolation control method according to the third invention is a biological sample isolation control method featuring the biological sample isolation control device that controls rotation of a rotating device in which a biological sample isolation container is placed according to the first invention, the method comprising a first step and a second step. In the first step, the drive unit of the rotating device is rotated at a first speed so that the biological sample and the reagent are layered in a state of having been brought closer to the outer surface in the radial direction in the isolation chamber. In the second step, the drive unit is rotated at a second rotation speed that is higher than the first speed to centrifuge the biological sample and the reagent.

The biological sample isolation control program according to the fourth invention causes a computer to execute the biological sample isolation control method according to the third invention.

### ADVANTAGEOUS EFFECTS

With the biological sample isolation container of the present invention, cells to be detected enable the proper isolation and collection without reducing the bioavailability of the cells.

### BRIEF DESCRIPTION OF DRAWINGS

Fig. 1 is an overall oblique view of the configuration of a biological sample isolation device in which is installed a biological sample isolation control device according to an embodiment of the present invention;
Fig. 2 is an oblique view of the configuration of the biological sample isolation device in Fig. 1 when a tray section has been opened;
Fig. 3 is an exploded oblique view of the configuration of the biological sample isolation device in Fig. 1;
Fig. 4 is a control block diagram of the biological sample isolation device in Fig. 1;
Fig. 5 is a plan view of the configuration of a container used for isolating and removing a mononuclear cell layer under rotation by the biological sample isolation device in Fig. 1 and in a state in which blood and a specific gravity adjusting agent have been layered;
Fig. 6 is an exploded oblique view of the container in Fig. 5;
Fig. 7 is a cross-sectional view of the container in Fig. 5;
Fig. 8A is a side cross-sectional view showing the position of the gas-liquid interface of the liquid at a stepped portion provided to the isolation chamber when the container in Fig. 5 is rotated at high speed, and Fig. 8B is a side cross-sectional view showing the position of the gas-liquid interface of the liquid at the stepped portion provided to the isolation chamber when the container of Fig. 5 is rotated at low speed;
Figs. 9A and 9B are side cross-sectional views showing the position of the gas-liquid interface of the liquid in the isolation chamber when the container is rotated at high speed and low speed, without the stepped portion of Figs. 8A and 8B;
Fig. 10 is a graph of the relation between the rotation speed and the elapsed time from the treatment of by rotating the container in Fig. 5 to apply centrifugal force using the biological sample isolation device in Fig. 1 and layer the blood and the specific gravity adjuster, up to the treatment of isolating the mononuclear cell layer;
Figs. 11A, 11B, 11C, and 11D are plan views showing the state inside the container when the container in Fig. 5 is rotated by the biological sample isolation control device in Fig. 1, and the blood and specific gravity adjuster are layered in the isolation chamber and centrifuged;
Figs. 12A, 12B, 12C, and 12D are plan views showing the state inside the container when the mononuclear cell layer, plasma, and some of the specific gravity adjuster are sent to the collection chamber from the liquid centrifuged by rotating the container in Fig. 5 with the biological sample isolation control device in Fig. 1, and the suspension other than the mononuclear cell layer is sent to the waste liquid chamber resuspension;
Fig. 13A is a graph of the relation between the rotation speed and the elapsed time when the container in Fig. 5 is rotated to suspend the liquid, and Fig. 13B is a graph of the relation between the acceleration in the radial direction and the circumferential direction and the elapsed time when the container in Fig. 5 is rotated to suspend the liquid;
Fig. 14A is a plan view of the container showing the state after centrifugation in the isolation chamber when the hematocrit rate in the blood is 35%, and Fig. 14B is a plan view of the container showing the state after centrifugation in the isolation chamber when the hematocrit rate in the blood is 50%;
Fig. 15A is a graph comparing the component ratios in 30 mL of liquid when the hematocrit ratio in blood is 35% and 50%, and Fig. 15B is a graph of the difference in the ratio accounted for by the specific gravity adjusting agent in the liquid containing the mononuclear cell layer, in a conventional comparative example and when the container in Fig. 5 is used;
Fig. 16 is a plan view of the configuration of the container according to another embodiment of the present invention;
Figs. 17A, 17B, 17C, and 17D are plan views showing the state inside the container when the container in Fig. 16 is rotated by the biological sample isolation control device in Fig. 1, and blood and a specific gravity adjusting agent are layered in the isolation chamber and centrifuged;
Figs. 18A, 18B, and 18C are plan views showing the state inside the container when red blood cells and mononuclear cells settle over time from diluted blood due to centrifugation when the container in Fig. 16 is rotated by the biological sample isolation control device in Fig. 1;
Fig. 19A is a plan view of the configuration of a container for isolating platelets in a collection solution from the state shown in Fig. 12D, and Fig. 19B is a plan view showing the position of a cleaning solution introduction hole that communicates with the cleaning solution chamber shown in Fig. 19A;
Figs. 20A, 20B, 20C, and 20D are plan views of a container and illustrate the step of isolating platelets in a collection liquid from the state shown in Fig. 19A; and
Fig. 21 is a graph of the relation between the elapsed time and the change in rotation speed of the container corresponding to the steps shown in Figs. 20A to 20D.

### DESCRIPTION OF EMBODIMENTS

### Embodiment 1

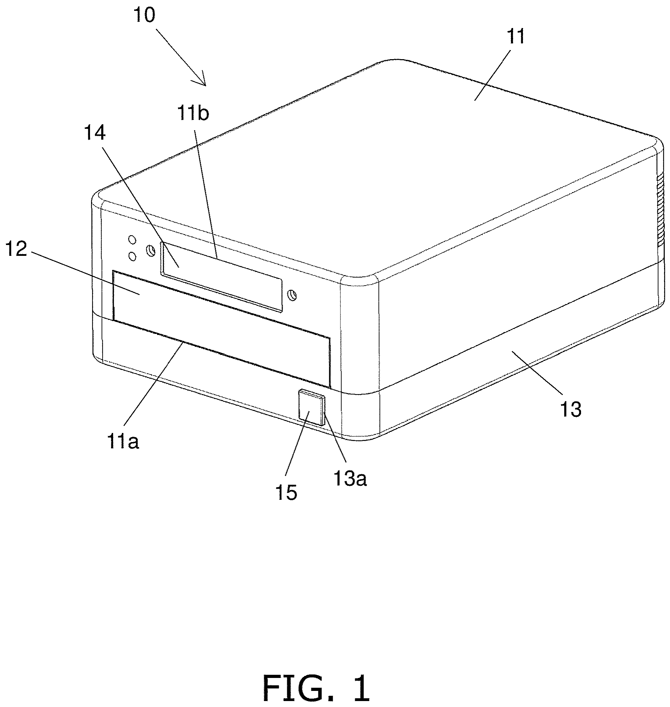

The biological sample isolation device 10 equipped with a biological sample isolation container (container 30) and a biological sample isolation control device (control unit 20), and the biological sample isolation control method according to an embodiment of the present invention will now be described with reference to Figs. 1 to 15B and Figs. 19 to 21.

In this embodiment, some unnecessarily detailed description may be omitted. For example, detailed description of already known facts or redundant description of components that are substantially the same may be omitted. This is to avoid unnecessary repetition in the following description, and facilitate an understanding on the part of a person skilled in the art.

The applicant has provided the appended drawings and the following description so that a person skilled in the art might fully understand this disclosure, but does not intend for these to limit what is discussed in the patent claims. (1) Configuration of Biological Sample Isolation Device 10

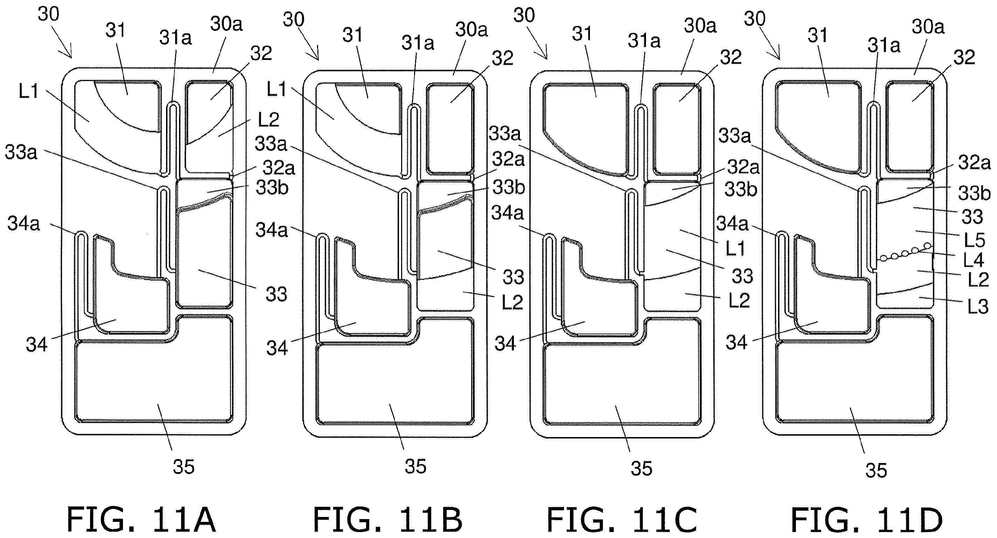

The biological sample isolation device (rotating device) 10 of this embodiment is a centrifuge that is used to layer blood (biological sample) L1 (see Fig. 11A) with a specific gravity adjusting agent (reagent) L2 (see Fig. 11A) in a container (biological sample isolation container) 30 without any mixing, and to efficiently extract a specific component (mononuclear cell layer L4) contained in the blood L1.

In this embodiment, the blood L1 is diluted blood to which physiological saline has been added.

With the biological sample isolation device 10 of this embodiment, first, the control unit 20 controls the rotation of the container 30 by the biological sample isolation device 10 so as to produce a state in which the blood L1 and the specific gravity adjusting agent L2 are appropriately layered inside the container 30.

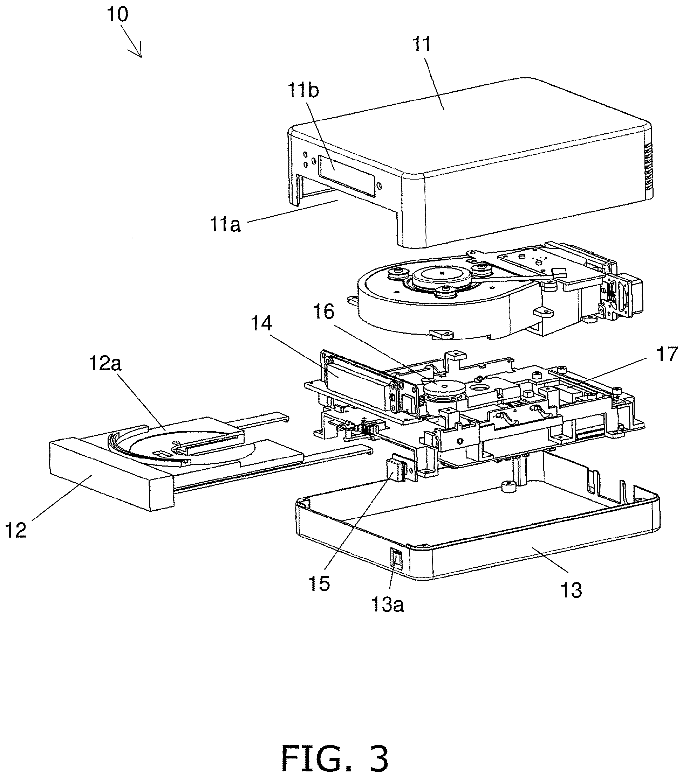

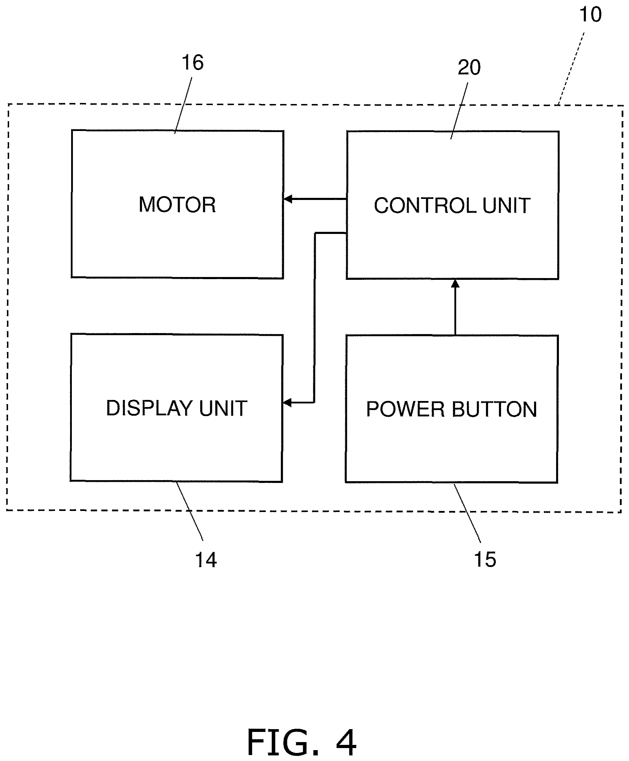

As shown in Fig. 1, the biological sample isolation device 10 is provided with a housing 11, a tray section 12, a base member 13, a display unit 14, a power button 15, a motor (rotational drive unit) 16 (see Fig. 3), a substrate unit 17 (see Fig. 3), and a control unit (biological sample isolation control device, rotational control unit) 20 (see Fig. 4).

As shown in Fig. 1, the housing 11 is a box-shaped member that forms the upper outer shell of the biological sample isolation device 10, and is provided on its front side with an opening 11a through which the tray section 12 moves in and out, and an opening 11b through which the display unit 14 is exposed.

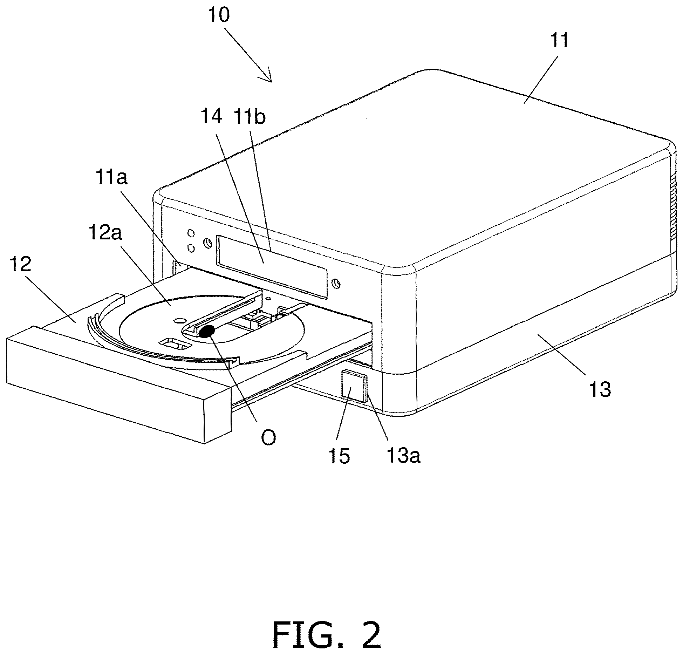

As shown in Fig. 1, the tray section 12 is attached in a state that allows it to be housed inside the housing 11, and as shown in Fig. 2, it moves to a state of protruding to the outside of the housing 11 under the drive force of a motor (not shown).

As shown in Fig. 2, the container 30 is placed in a container holder 12a provided on the upper surface of the tray section 12 in a state in which the tray section 12 has been deployed to the outside of the housing 11.

In the container holder 12a, containers 30 are put in place so that a plurality of them are disposed radially around a rotation center O (see Fig. 2, etc.), for example. Consequently, when the containers 30 are rotated, centrifugal force is applied in a radially outward direction to the blood L1, etc., held in the containers 30.

As shown in Figs. 1 and 2, the base member 13 is a box-shaped member that combines with the housing 11 to form the outer shell of the biological sample isolation device 10, and is provided at the bottom part of the housing 11. As shown in Fig. 3, the front surface of the base member 13 is provided with an opening 13a that exposes the power button 15 (discussed below).

As shown in Figs. 1 and 2, the display unit 14 is provided so as to be exposed on the front side of the housing 11, and displays various kinds of information when the power button 15 is pushed to switch on the biological sample isolation device 10. Also, the display unit 14 is provided at the end of the substrate unit 17 as shown in Fig. 3, and is controlled by the control unit 20 (see Fig. 4) included in the substrate unit 17.

As shown in Fig. 1, the power button 15 is provided so as to be exposed on the front side of the base member 13, and is pushed to switch the power to the biological sample isolation device 10 on and off. As shown in Fig. 3, the power button 15 is provided at the end of the substrate unit 17, similar to the display unit 14, and the operation of turning the power on or off is inputted to the control unit 20 (see Fig. 4) included in the substrate unit 17.

As shown in Fig. 3, the motor 16 is provided inside the housing 11 and rotates the containers 30. The motor 16 rotates the containers 30 placed in the container holder 12a of the tray section 12 in a specific rotational direction at a specific rotational speed, thereby applying centrifugal force to the blood L1. etc., stored in the containers 30.

A biological sample isolation control method featuring rotation control of the containers 30 by the motor 16 will be described in detail below.

As shown in Fig. 3, the substrate unit 17 is attached to the upper surface of the base member 13, and the tray section 12 is attached in a state of being able to be deployed and retracted. The substrate unit 17 is electrically connected to the above-mentioned display unit 14 and power button 15, and includes a control board that controls these components.

The control unit (biological sample isolation control device, control unit) 20 includes a CPU and a motor driver IC provided on an electric board attached to the underside of the substrate unit 17 shown in Fig. 3. The control unit 20 is connected to the display unit 14, the power button 15, and the motor 16 as shown in Fig. 4. The control unit 20 switches the power state of the device when the power button 15 is pushed. Also, the control unit 20 controls the display on the display unit 14 and controls the rotation of the motor 16.

### (2) Configuration of Containers 30

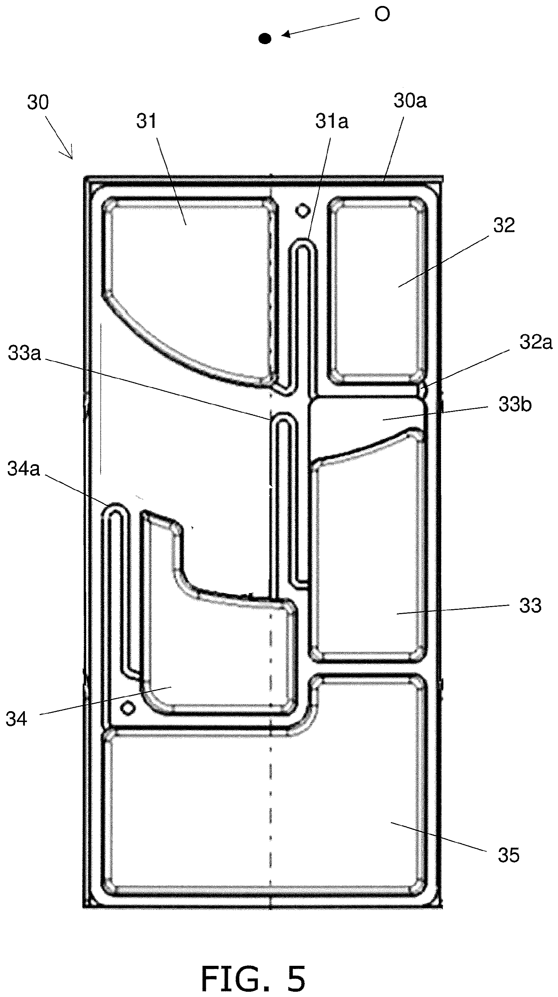

The containers (biological sample isolation containers) 30 used to isolate the blood L1 component in the biological sample isolation device 10 of this embodiment will now be described with reference to Figs. 5 to 7.

As shown in Fig. 5, a container 30 includes a main body portion 30a in which blood (biological sample) L1 and a specific gravity adjusting agent (reagent) L2 are sealed, a specimen chamber 31 for storing the blood L1, a reagent chamber 32 for storing the specific gravity adjusting agent L2, an isolation chamber 33 in which the blood L1 and the specific gravity adjusting agent L2 are centrifuged to isolate them into their components, a siphon flow path (third siphon flow path) 31a that connects the specimen chamber 31 and the isolation chamber 33, a capillary flow path 32a that connects the reagent chamber 32 and the isolation chamber 33, a collection chamber 34 that stores and collects the mononuclear cell layer L4 from among the components subjected to centrifugal isolation, a waste liquid chamber 35 that stores the supernatant liquid suspended in the collection chamber 34 from among the components subjected to centrifugal isolation, a siphon flow path (first siphon flow path) 33a that connects the isolation chamber 33 and the collection chamber 34, and a siphon flow path (second siphon flow path) 34a that connects the collection chamber 34 and the waste liquid chamber 35.

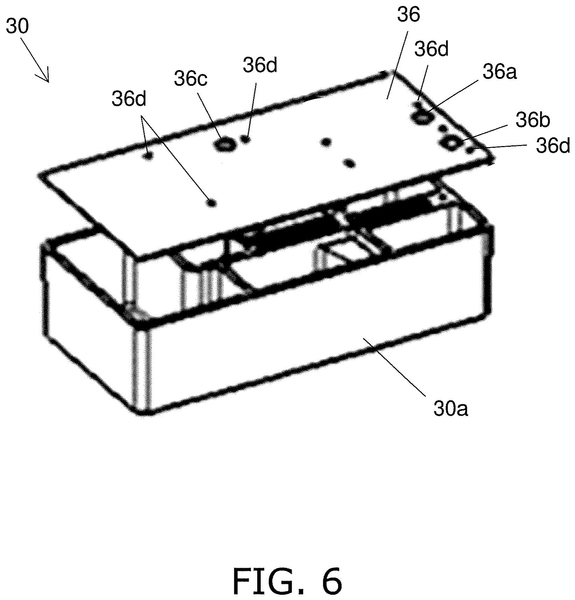

The specimen chamber 31 holds the blood L1 introduced through an introduction hole (first introduction hole) 36a (see Fig. 6; discussed below).

As shown in Fig. 5, the siphon flow path (third siphon flow path) 31a is disposed to the inside in the radial direction of the isolation chamber 33 around the rotation center O, connects the specimen chamber 31 holding the blood L1 with the isolation chamber 33, and utilizes a siphon effect to send the blood L1 from the specimen chamber 31 to the isolation chamber 33 so as to be layered on the specific gravity adjusting agent L2.

A first end of the siphon flow path 31a is connected to a portion of the side wall surface constituting the specimen chamber 31 near the intersection of a surface extending in the radial direction centered on the rotation center O and a surface intersecting the radial direction. A second end on the opposite side from the first end is connected to a surface on the inside diameter side and intersecting the radial direction of the side wall constituting the isolation chamber 33.

Also, the siphon flow path 31a is formed between a recess formed in the upper surface of the main body portion 30a of the container 30 and the lid member 36.

The siphon flow path 31a is disposed on the upper side in the vertical direction in which gravity acts on the liquid when liquid is held in the specimen chamber 31 and the isolation chamber 33, and connects the specimen chamber 31 and the isolation chamber 33 on the upper side in the vertical direction.

Consequently, when the containers 30 are not rotating, that is, when no centrifugal force is being applied to the blood L1, the blood L1 is held by gravity on the bottom side of the specimen chamber 31, and when centrifugal force is applied, the blood L1 accumulates to the outside in the radial direction in the specimen chamber 31, reaching the connected part of the siphon flow path (third siphon flow path) 31a.

The reagent chamber 32 holds a specific gravity adjusting agent L2 introduced through an introduction hole (second introduction hole) 36b (see Fig. 6; discussed below).

As shown in Fig. 5, the capillary flow path 32a is disposed to the inside in the radial direction of the isolation chamber 33 around the rotation center O, and connects the reagent chamber 32, which holds the specific gravity adjusting agent L2, to the isolation chamber 33. The capillary flow path 32a utilizes capillary action to send the specific gravity adjusting agent L2 from the reagent chamber 32 to the isolation chamber 33 ahead of the blood L1.

A first end of the capillary flow path 32a is connected to an outer surface in the radial direction centered on the rotation center O, among the side wall surfaces constituting the reagent chamber 32. A second end on the opposite side from the first end is connected to an inner surface intersecting the radial direction, among the side wall surfaces constituting the isolation chamber 33.

Also, the capillary flow path 32a is disposed on the upper side in the vertical direction in which gravity is applied to the specific gravity adjusting agent when the specific gravity adjusting agent L2 is stored in the reagent chamber 32, and connects the reagent chamber 32 and the isolation chamber 33 on the upper side in the vertical direction. The capillary flow path 32a is formed between a recess formed in the upper surface of the main body portion 30a and the lid member 36.

Consequently, when the container 30 is not rotating, that is, when no centrifugal force is applied to the specific gravity adjusting agent L2, the specific gravity adjusting agent L2 is held by gravity on the bottom side of the reagent chamber 32, and when centrifugal force is applied, the specific gravity adjusting agent L2 accumulates on to the outside in the radial direction of the reagent chamber 32 and reaches the connected portion of the capillary flow path 32a.

The isolation chamber 33 holds the blood L1 sent from the specimen chamber 31 and the specific gravity adjusting agent L2 sent from the reagent chamber 32 in a layered state, and rotates the containers 30 to apply centrifugal force, which causes the components that have been centrifuged to be held within the isolation chamber 33.

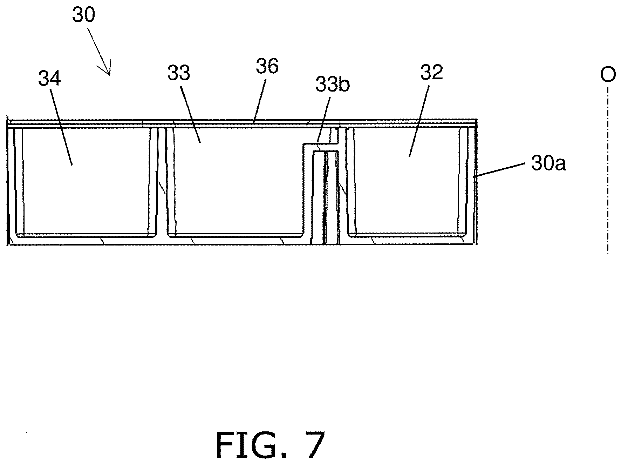

Furthermore, as shown in Fig. 7, the isolation chamber 33 has a stepped portion 33b, which is shorter in the vertical direction than at a position to the outside in the radial direction, at a position on the inside in the radial direction centered on the rotation center O.

As shown in Fig. 5, the stepped portion 33b is provided at the portion of the isolation chamber 33 where the siphon flow path 31a, which sends blood L1 from the specimen chamber 31 to the isolation chamber 33, and the capillary flow path 32a, which sends specific gravity adjusting agent L2 from the reagent chamber 32 to the isolation chamber 33, are connected.

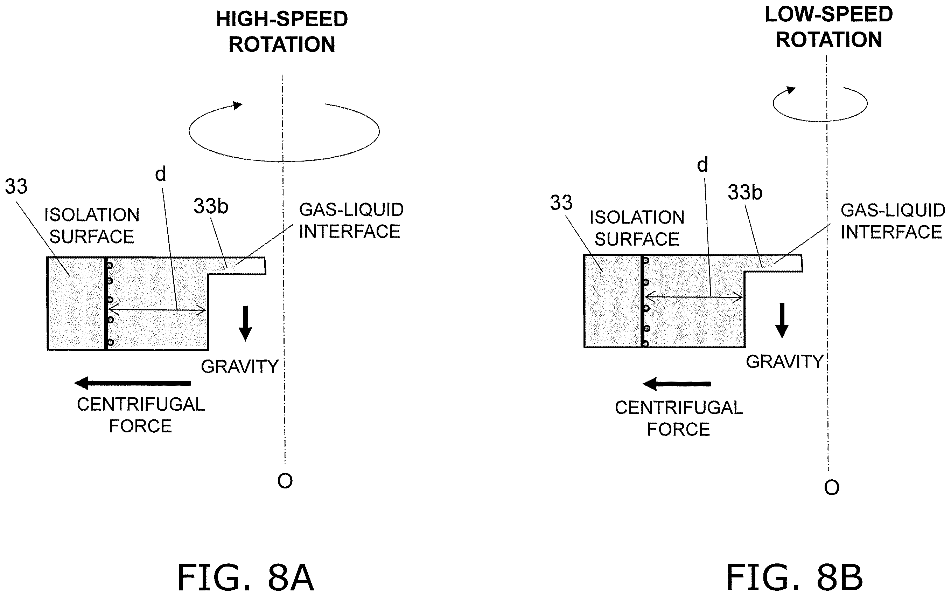

As shown in Figs. 8A and 8B, the stepped portion 33b is provided at a position in the isolation chamber 33 that is filled with the liquid obtained by centrifugal isolation processing of blood L1 and specific gravity adjusting agent L2 under the application of centrifugal force.

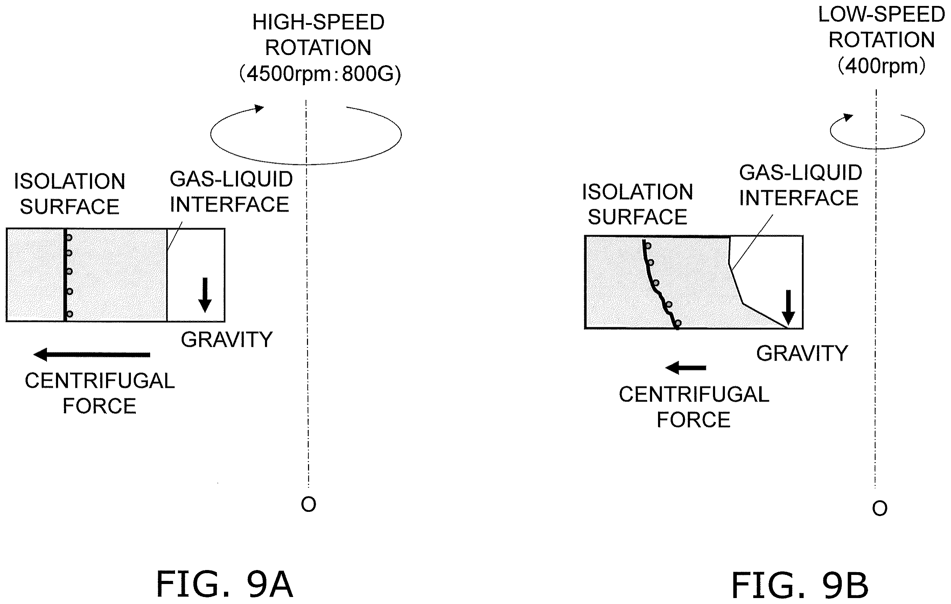

Here, if the stepped portion 33b is not provided, as shown in Fig. 9A, when the container is rotated at high speed (such as 4500 rpm) and a centrifugal force of about 800 G is applied to the liquid in the container, the liquid will hardly be affected by gravity and the gas-liquid interface of the liquid will be stable. Therefore, the isolation surface of the specific component subjected to the centrifugal isolation will also be stable.

On the other hand, as shown in Fig. 9B, when the container is rotated at a low speed (such as 400 rpm) and the centrifugal force acting on the liquid in the container is reduced to about 4 G, the liquid will be affected by gravity, and the gas-liquid interface of the liquid will become unstable, and therefore the isolation surface of a specific component processed by centrifugation will also become unstable.

With the container 30 in this embodiment, the stepped portion 33b that reduces the size of the container 30 in the depth direction is provided at a position to the inside in the radial direction centered on the rotation center O in the isolation chamber 33.

As shown in Figs. 8A and 8B, the gas-liquid interface of the liquid is smaller in the depth direction at the position of stepped portion 33b than at other positions in isolation chamber 33. This effectively prevents the isolation surface from becoming unstable when the mononuclear cell layer L4 is taken out due to instability of the gas-liquid interface of the liquid in isolation chamber 33 under the effect of gravity when the rotation speed of containers 30 decreases.

As a result, the mononuclear cell layer L4 can be efficiently sent to the collection chamber 34 and removed from the containers 30.

Also, as shown in Fig. 8A, the vertical depth of the stepped portion 33b is smaller than the distance d between the radial isolation position at which the mononuclear cell layer L4 contained in the liquid obtained by centrifuging the specific gravity adjusting agent L2 and blood L1 is located, and the radial inner wall surface of the isolation chamber 33.

This effectively prevents the isolation surface from becoming unstable when taking out the mononuclear cell layer L4, even if the gas-liquid interface of the liquid in the isolation chamber 33 becomes unstable due to the effect of gravity when the rotation speed of the containers 30 decreases.

Here, it is desirable for the stepped portion 33b provided to the isolation chamber 33 to be optimized in consideration of the shape of the isolation chamber 33 and the rotation speed of the containers 30.

For example, if the isolation chamber 33 has a width of 9.8 mm, a length of 17.8 mm, and a depth of 14 mm, and the rotation speed of the containers 30 for connecting the siphon flow path 33a is 400 rpm, then the depth of the stepped portion 33b is preferably 2 mm.

When the rotation speed of the container 30 is 400 rpm, the liquid in the isolation chamber 33 is subjected to a centrifugal force of about 4 G acting outward in the radial direction and to gravitational acceleration of 1 G acting downward.

At this point, because the stepped portion 33b is provided to the isolation chamber 33 as mentioned above, the gas-liquid interface is less likely to become unstable when the liquid is subjected to the effect of gravity, and the isolation surface of the specific component (mononuclear cell layer L4) can also be kept in a stable state.

In terms of maintaining the gas-liquid interface, it is effective to reduce the depth of stepped portion 33b, but if the depth is reduced too much, the capacity of stepped portion 33b will be small, and therefore when filling up to the stepped portion 33b with liquid, if there is any variance in the amount of liquid, the liquid may not reach stepped portion 33b, or may go past the stepped portion 33b.

With the containers 30 in this embodiment, the volume at the stepped portion 33b is about 3% of the volume of the isolation chamber 33.

As shown in Fig. 5, the siphon flow path (first siphon flow path) 33a is disposed on the inside of the collection chamber 34 in the radial direction centered on the rotation center O, and connects the isolation chamber 33 and the collection chamber 34. The siphon flow path 33a uses siphoning to send the centrifuged components (mononuclear cell layer L4, the plasma L5, and some of the specific gravity adjusting agent L2) held in the isolation chamber 33 from the isolation chamber 33 to the collection chamber 34.

The first end of the siphon flow path 33a is connected to a surface running in the radial direction centered on the rotation center O of the side wall surface constituting the isolation chamber 33, and the second end on the opposite side from the first end is connected to a surface on the inside diameter side intersecting the radial direction of the side wall surface constituting the collection chamber 34.

The siphon flow path 33a connects the isolation chamber 33 and the collection chamber 34 on the upper side in the vertical direction. The siphon flow path 33a is disposed on the upper side in the vertical direction in which gravity acts on the liquid when the liquid is held in the isolation chamber 33.

The first end of the siphon flow path 33a is connected at a position on the outside in the radial direction to the portion of the side wall surface of the isolation chamber 33 where the centrifuged mononuclear cell layer L4 is located (see Fig. 11D).

Consequently, components with low specific gravity, including the mononuclear cell layer L4, can be sent to the collection chamber 34 by applying centrifugal force in a state in which the components subjected to centrifugal isolation have reached the part of the siphon flow path 33a that is connected to the isolation chamber 33.

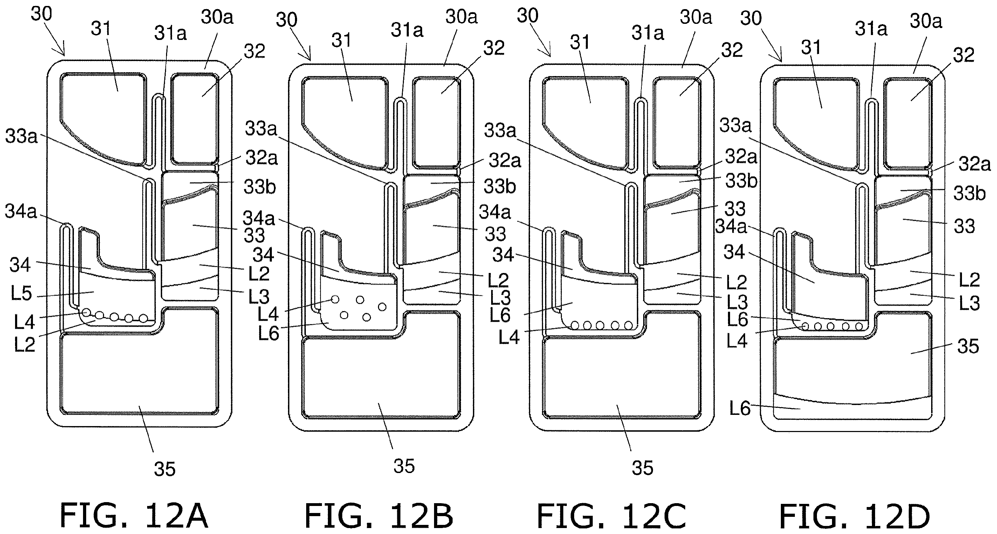

As shown in Fig. 5, the collection chamber 34 is provided at a position to the outside in the radial direction of the specimen chamber 31 and the reagent chamber 32, and slightly to the outside in the radial direction of the isolation chamber 33. The collection chamber 34 holds the mononuclear cell layer L4, some of the specific gravity adjusting agent L2, and the plasma L5 contained in the blood L1, which are sent from the isolation chamber 33 and centrifuged in the isolation chamber 33 (see Fig. 12A). After these have been suspended, the collection chamber 34 causes just the mononuclear cell layer L4 to settle outward in the radial direction in which the centrifugal force is applied in the liquid containing the mononuclear cell layer L4, some of the specific gravity adjusting agent L2, and the plasma L5 contained in the blood L1 (see Fig. 12C).

As shown in Fig. 5, the siphon flow path 34a connects the collection chamber 34 and the waste liquid chamber 35, and uses siphoning to send the supernatant (plasma L5 and some of the specific gravity adjusting agent L2) of the mononuclear cell layer L4 that has settled after suspension in the collection chamber 34 from the collection chamber 34 to the waste liquid chamber 35.

The first end of the siphon flow path 34a is connected to a surface running in the radial direction centered on the rotation center O of the side wall surface constituting the collection chamber 34, and the second end on the opposite side from the first end is connected to the surface on the inside diameter side intersecting the radial direction, of the side wall surface constituting the waste liquid chamber 35.

The siphon flow path 34a connects the collection chamber 34 and the waste liquid chamber 35 on the upper side in the vertical direction. The siphon flow path 34a is disposed on the upper side in the vertical direction in which gravity acts on the liquid when the liquid is held in the collection chamber 34. The siphon flow path 34a connects the collection chamber 34 and the waste liquid chamber 35 on the upper side in the vertical direction.

As shown in Fig. 6, the container 30 of this embodiment further includes a lid member 36 that is attached so as to cover the upper surface of the main body portion 30a on whose upper surface are provided the isolation chamber 33, the collection chamber 34, the waste liquid chamber 35, the siphon flow path 33a, and the siphon flow path 34a.

Consequently, the siphon flow path 31a, the capillary flow path 32a, the siphon flow path 33a, and the siphon flow path 34a are formed between the lid member 36 and a recess formed in the upper surface of the main body portion.

The lid member 36 has an introduction hole (first introduction hole) 36a for introducing the blood L1, an introduction hole (second introduction hole) 36b for introducing the specific gravity adjusting agent L2, a collection hole 36c for collecting the mononuclear cell layer L4, and a plurality of air holes 36d.

Consequently, when the blood L1 is introduced through the introduction hole 36a into the specimen chamber 31, the blood L1 can fill the specimen chamber 31 while air inside the specimen chamber 31 is removed through the air holes 36d. Similarly, when the specific gravity adjusting agent L2 is introduced through the introduction hole 36b into the reagent chamber 32, the specific gravity adjusting agent L2 can fill the reagent chamber 32 while air inside the reagent chamber 32 is removed through the air holes 36d. Furthermore, when liquid containing the mononuclear cell layer L4 in the collection chamber 34 is extracted from the collection hole 36c, the liquid containing the mononuclear cell layer L4 can be collected while air is brought into the collection chamber 34 through the air holes 36d. Biological Sample Isolation Control Method

As discussed above, the biological sample isolation device 10 of this embodiment is equipped with a control unit (biological sample isolation control device, rotation control unit) 20 (see Fig. 4) that controls the rotation of the motor 16 used to rotate the containers 30.

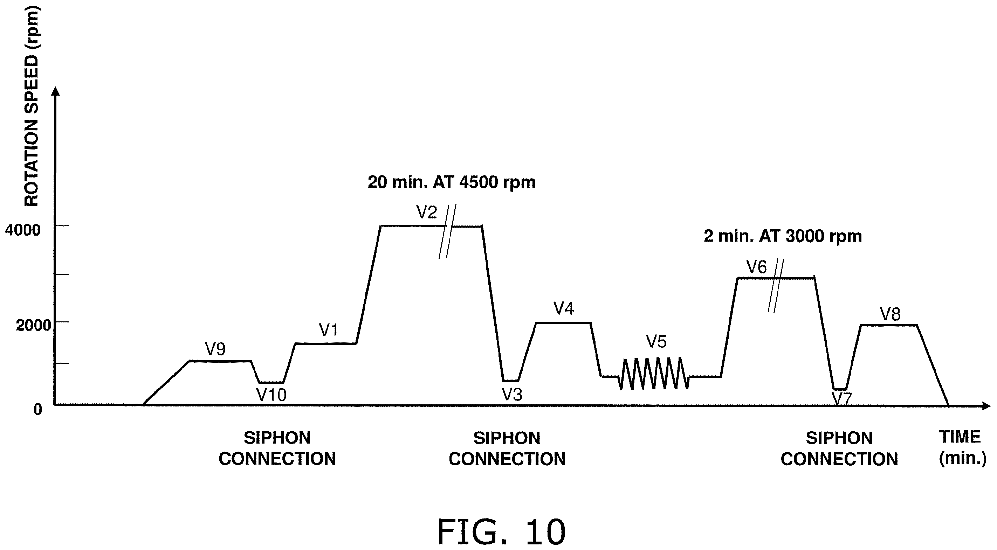

As shown in Fig. 10, the control unit 20 controls the motor 16 so as to layer the blood L1 and the specific gravity adjusting agent L2 contained in the containers 30 while gradually changing the rotation speed of the motor 16, to subject these to centrifugal isolation, and to isolate and extract a specific component (mononuclear cell layer L4).

That is, the control unit 20 controls the rotation of the motor 16 so that the blood L 1 held in the specimen chamber 31 and the specific gravity adjusting agent L2 held in the reagent chamber 32 are layered in the isolation chamber 33.

At this point, when the control unit 20 rotates the motor 16 at a speed V9 (see Fig. 10) to apply centrifugal force to the containers 30, as shown in Fig. 11A, the specific gravity adjusting agent L2 held in the reagent chamber 32 is sent to the isolation chamber 33 before the blood L1 held in the specimen chamber 31 (see Fig. 11B).

The introduced blood L1 remains in the specimen chamber 31 even when centrifugal force is applied, because the outlet of the specimen chamber 31 is connected to the siphon flow path 31a. On the other hand, the specific gravity adjusting agent L2 is sent to the isolation chamber 33 when the centrifugal force becomes greater than the capillary force in the capillary flow path 32a, because the outlet of the reagent chamber 32 is connected to the capillary flow path 32a.

Next, as shown in Fig. 11C, after all of the specific gravity adjusting agent L2 has been sent to the isolation chamber 33, the control unit 20 gradually lowers the rotation speed of the motor 16 to a speed V10 (see Fig. 10) until the capillary force becomes greater than the centrifugal force, and the blood L1 fills the siphon flow path 31a at the outlet of the specimen chamber 31. After this, the control unit 20 gradually raises the rotation speed of the motor 16 to a speed V1 (see Fig. 10), and the blood L1 in the specimen chamber 31 starts moving to the isolation chamber 33 and is slowly layered on top of the specific gravity adjusting agent L2.

At this point, the siphon flow path 33a is connected to a certain inner peripheral position from the outer periphery of the isolation chamber 33, but above a certain rotation speed, the liquid is retained as it is within the isolation chamber 33 and is not sent out through the siphon flow path 33a.

Next, after the blood L1 has been layered on the specific gravity adjusting agent L2, the control unit 20 gradually raises the rotation speed of the motor 16 to a speed V2 (see Fig. 10), and performs centrifugal isolation for about 20 minutes at about 800 G. As a result, as shown in Fig. 11D, red blood cells L3, the specific gravity adjusting agent L2, the mononuclear cell layer (specific component) L4, and the plasma L5 are isolated in order of decreasing specific gravity from the outside in the radial direction of the isolation chamber 33.

Next, after the centrifugation, the control unit 20 lowers the rotation speed of the motor 16 to a speed V3 (see Fig. 10), and once the capillary force becomes greater than the centrifugal force, the siphon flow path 33a at the outlet of the isolation chamber 33 is filled with liquid.

The speed V3 at which the liquid fills the siphon flow path 33a connected to the isolation chamber 33 is lower than the speed V10 at which the blood L1 fills the siphon flow path 31a of the specimen chamber 31.

After this, when the rotation speed is gradually raised to a speed V4 (see Fig. 10), as shown in Fig. 12A, the liquid located higher than (to the inside in the radial direction) the connection portion of the siphon flow path 33a of the isolation chamber 33 is sent from the isolation chamber 33 to the collection chamber 34.

At this point, the position where the siphon flow path 33a is connected to the isolation chamber 33 is slightly more to the outside in the peripheral direction (the outside in the radial direction) than the position of the mononuclear cell layer L4 after the centrifugation.

This keeps the amount of specific gravity adjusting agent L2 sent into the collection chamber 34 to a minimum. As a result, this effectively prevents a decrease in the bioavailability of the cells contained in the mononuclear cell layer L4.

Next, the control unit 20 repeatedly raises and lowers the rotational speed of the motor 16 in a speed range V5 (see Fig. 10) that is lower than the speed V4, thereby suspending the mononuclear cell layer L4, the specific gravity adjusting agent L2, and the plasma L5 in the collection chamber 34 to produce a suspension L6, as shown in Fig. 12B.

Next, the control unit 20 raises the rotational speed of the motor to a speed V6 (see Fig. 10) to apply centrifugal force, causing the mononuclear cell layer L4 to settle to the bottom (outer surface in the radial direction) of the collection chamber 34, as shown in Fig. 12C.

Next, the control unit 20 lowers the rotation speed of the motor 16 to a speed V7 (see Fig. 10) to fill the outlet (siphon flow path 34a) of the collection chamber 34 with the supernatant of the suspension L6 other than the mononuclear cell layer L4, and then raises the speed to a speed V8 (see Fig. 10). This allows the supernatant of the mononuclear cell layer L4 to be sent to the waste liquid chamber 35 via the siphon flow path 34a, as shown in Fig. 12D.

Next, the control unit 20 halts the rotation of the motor 16, allowing the liquid containing the mononuclear cell layer L4 that remains in the collection chamber 34 to be easily taken out through the collection hole 36c.

As discussed above, in this embodiment, the mononuclear cell layer L4, which is the specific component to be isolated and collected, can be removed from the collection chamber 34 while minimizing the adverse effects caused by mixing with the specific gravity adjusting agent L2.

The rotation control mode used by the control unit 20 will now be described.

The control unit 20 rotates the motor 16 at a speed V9 that is lower than the speed V1, and sends the specific gravity adjusting agent L2 from the reagent chamber 32 to the isolation chamber 33 through the capillary flow path 32a (see Fig. 11B) (ninth mode).

The control unit 20 rotates the motor 16 at a speed V10 that is lower than the speed V9, causing the blood L1 to flow into the siphon flow path 31a (tenth mode).

The control unit 20 rotates the motor 16 at the speed V1 so that the blood L1 and the specific gravity adjusting agent L2 are layered in the isolation chamber, with the blood L1 and the specific gravity adjusting agent L2 concentrated toward the outer surface in the radial direction (see Fig. 11C) (first mode).

Here, the control unit 20 rotates the motor 16 at the first speed V1 that is higher than the speed V10, and sends the blood L1 to the isolation chamber 33 via the siphon flow path 31a so that the blood L1 is layered on top of the specific gravity adjusting agent L2 (eleventh mode).

Then, the control unit 20 rotates the motor 16 at a speed V2 that is higher than the speed V1, to centrifuge the blood L1 and the specific gravity adjusting agent L2 (see Fig. 11D) (second mode).

Also, after the blood L1 and the specific gravity adjusting agent L2 have been centrifuged in the second mode, the control unit 20 rotates the motor 16 at a speed V3 that is lower than the speeds V1 and V2, thereby causing the liquid containing the mononuclear cell layer L4 to flow into the siphon flow path 33a (third mode).

After the third mode, the control unit 20 rotates the motor 16 at a speed V4 that is higher than the speed V3, and sends the liquid containing the mononuclear cell layer L4 from the isolation chamber 33 to the collection chamber 34 (see Fig. 12A) (fourth mode).

Also, after the fourth mode, the control unit 20 raises or lowers the rotation speed in a speed range V5 that is lower than the speed V4 so as to suspend the liquid containing the mononuclear cell layer L4 sent to the collection chamber 34 (see Fig. 12B) (fifth mode).

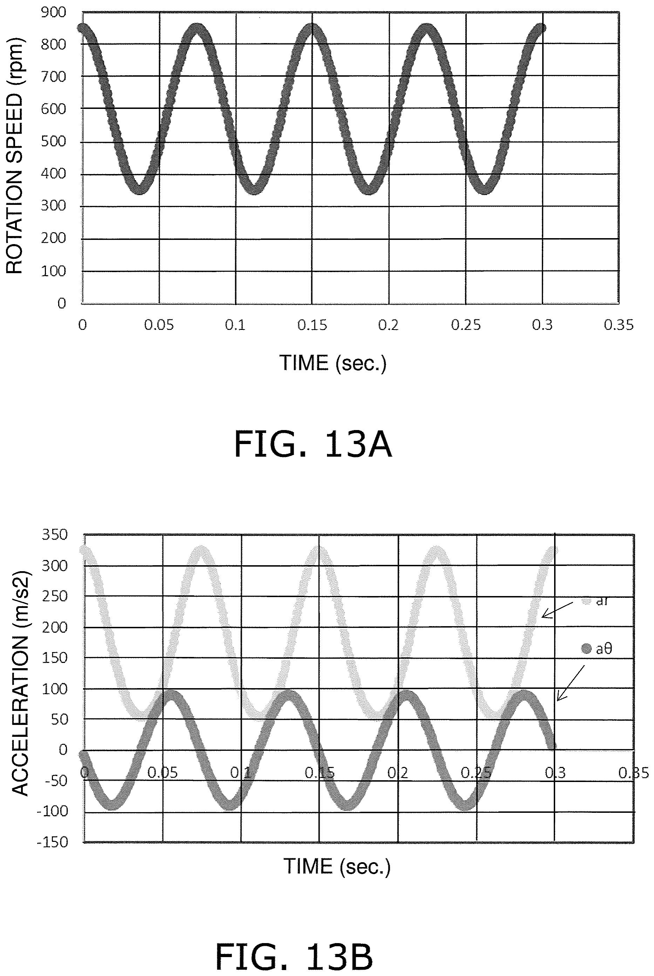

Here, the control unit 20 controls the rotation of the motor 16 so that its rotation speed is repeatedly raised and lowered within a speed range V5 of 350 to 850 rpm, as shown in Fig. 13A.

The suspension treatment requires that the liquid in the collection chamber 34 be mixed while the liquid is held in the collection chamber 34 by the outlet siphon flow path 34a, thereby mixing the specific gravity adjusting agent L2 and the plasma L5.

In this embodiment, the collection chamber 34 is used to hold liquid, measures 13 mm wide, 7.6 mm long, and 14 mm deep, and is located around the outer periphery about 40 mm from the rotation center O. The siphon flow path 34a is formed by a capillary tube having a width of 0.6 mm and a depth of 0.2 mm at the outlet of the collection chamber 34.

Here, at approximately 300 rpm (4 G) or less, the capillary force of the siphon flow path 34a becomes greater than the centrifugal force, so the connection portion of the siphon flow path 34a is filled, after which the rotation speed is raised, sending the liquid in the collection chamber 34 to the waste liquid chamber 35. Therefore, in order to retain the supernatant liquid in the collection chamber 34, it is necessary to maintain a rotation speed higher than 300 rpm.

Also, in the suspension treatment, the control unit 20 superimposes a basic rotation speed of the motor 16 of 600 rpm, a frequency of 13.4 Hz, and a rotation speed change of ±250 rpm for 40 seconds.

At this point, suddenly raising or lowering the rotation speed of the containers 30 generates a circumferential acceleration aθ as shown in Fig. 13B, and applies a force in the peripheral direction to the liquid in the containers 30, suspending the liquid.

Here, the minimum rotation speed of the motor 16 in the suspension treatment is the rotation speed at which the centrifugal force becomes greater than the capillary force of the siphon flow path 34a (for example, 300 rpm or more). Also, *ar* shown in Fig. 13B is the centrifugal force in the radial direction.

The control unit 20 controls the rotation of the motor 16 so that *aθ > ar,* which affords efficient suspension of the liquid. Also, raising or lowering the rotation speed at a frequency that matches the natural frequency of the collection chamber 34 results in an increase in disturbance of the liquid surface through resonance, and improves the suspension efficiency.

After the fifth mode, the control unit 20 rotates the motor 16 at a speed V6 that is higher than the speed range V5, to centrifuge the liquid containing the mononuclear cell layer L4, and cause the mononuclear cell layer L4 to settle on the radially outer surface of the collection chamber 34 (see Fig. 12C) (sixth mode).

By suspending the liquid in the collection chamber 34, some of the specific gravity adjusting agent L2 is mixed with the plasma L5, and the specific gravity becomes smaller than that of the mononuclear cell layer L4, so raising the rotation speed of the motor 16 allows the mononuclear cell layer L4 to settle to the surface on the outside in the radial direction of the collection chamber 34.

The centrifugation of the mononuclear cell layer L4 is performed for two minutes at 400 G, for example. In this embodiment, the control unit 20 sets the rotation speed of the motor 16 to about 3000 rpm, and performs centrifugation for two minutes, thereby causing the mononuclear cell layer L4 to settle on the surface on the outside in the radial direction of the collection chamber 34.

After the sixth mode, the control unit 20 rotates the motor 16 at a speed V7 that is lower than speed V6, causing the supernatant liquid (the plasma L5 and some of the specific gravity adjusting agent L2) excluding the mononuclear cell layer L4 to flow into the siphon flow path 34a (seventh mode).

After the seventh mode, the control unit 20 rotates the motor 16 at a speed V8 that is higher than the speed V7, and sends the supernatant liquid other than the mononuclear cell layer L4 (the plasma L5 and some of the specific gravity adjusting agent L2) from the collection chamber 34 to the waste liquid chamber 35 (see Fig. 12D) (eighth mode).

Since the quantity of red blood cells L3 contained in the blood L1 varies with the hematocrit value contained in the blood L1 being used, the radial position of the mononuclear cell layer L4 varies when centrifugal force is applied. Accordingly, the position where the siphon flow path 33a is connected to the isolation chamber 33 is set slightly more to the outside (in the radial direction) than the position of the mononuclear cell layer L4 after the centrifugal isolation, to match the conditions on the lower side of the range of the hematocrit value of the blood L1 being used.

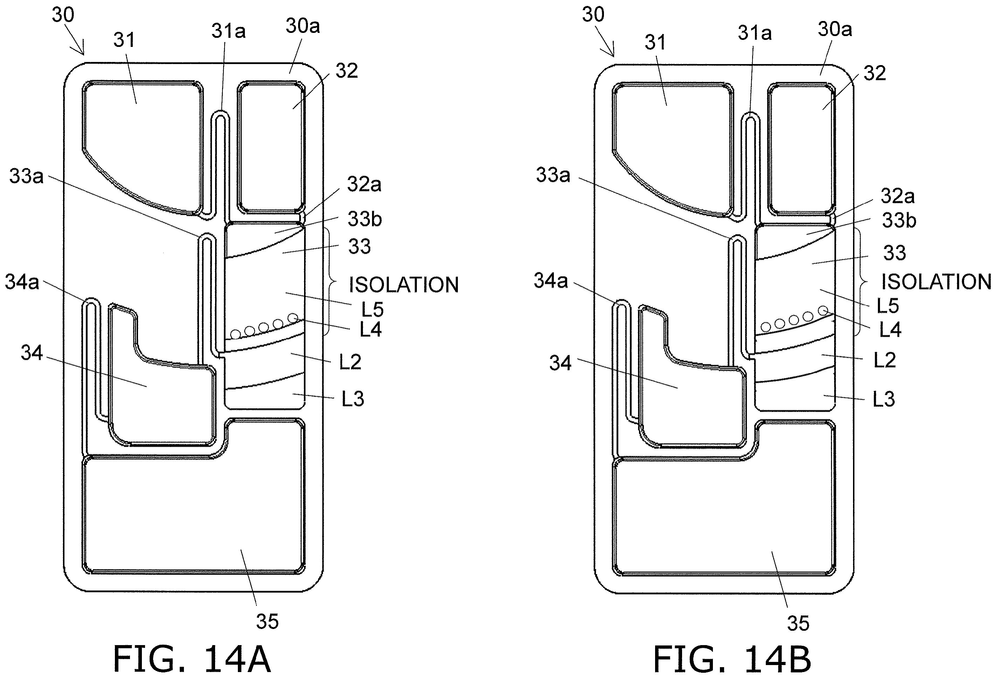

That is, when the hematocrit value of the blood L1 is greater than that of the blood L1 in Fig. 14A, as shown in Fig. 14B, a larger amount of the specific gravity adjusting agent L2 is isolated and sent into the collection chamber 34, but is sent along with all of the plasma L5.

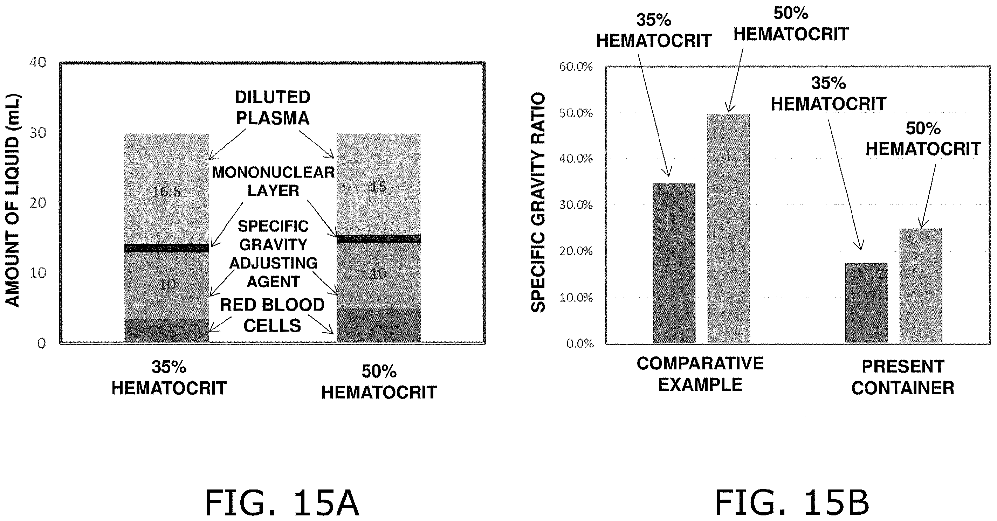

Consequently, as shown in Fig. 15A, for example, when the liquid volume distribution after centrifugation at each hematocrit value is examined using 10.0 mL of blood, 10.0 mL of diluent, and 10.0 mL of specific gravity adjusting agent, a comparison of the cases of 35% and 50% hematocrit in the blood L1 reveals that the amounts of plasma (diluted plasma) L5 contained in 10 mL of the blood L1 are 16.5 mL and 15.0 mL. The amount of specific gravity adjusting agent L2 is 10.0 mL in both cases. The amounts of red blood cells L3 are 3.5 mL and 5.0 mL.

In this case, a comparison with the configuration in a comparative example in which no plasma L5 component is sent to the collection chamber 34, as shown in Fig. 15B, the container 30 in this embodiment has a smaller ratio of specific gravity adjusting agent L2 than in the comparative example, regardless of whether the hematocrit is 35% or 50%.

Consequently, the mononuclear cell layer L4 is sent along with all of the plasma L5 and some of the specific gravity adjusting agent L2, which suppresses the adverse effect of the specific gravity adjusting agent L2 and effectively prevents a decrease in the bioavailability of the cells contained in the mononuclear cell layer L4. Isolation of Platelets Contained in Collected Liquid

In the state shown in Fig. 12D described above, that is, in a state in which the supernatant liquid other than the mononuclear cell layer L4 (the plasma L5 and some of the specific gravity adjusting agent L2) is sent from the collection chamber 34 to the waste liquid chamber 35 (eighth mode), many platelets end up remaining in the collection liquid left in the collection chamber 34. Accordingly, it is necessary to dilute the liquid collected from the container 30 with a 0.9% NaCl solution or the like in a centrifuge tube and then perform centrifugal isolation, which is a problem in that the operation becomes more complicated.

In view of this, in this embodiment, in the state shown in Fig. 12D, the configuration may be such that platelets are isolated from the collected liquid using a container 230 having a cleaning liquid chamber 236 into which a cleaning liquid L7 is introduced manually.

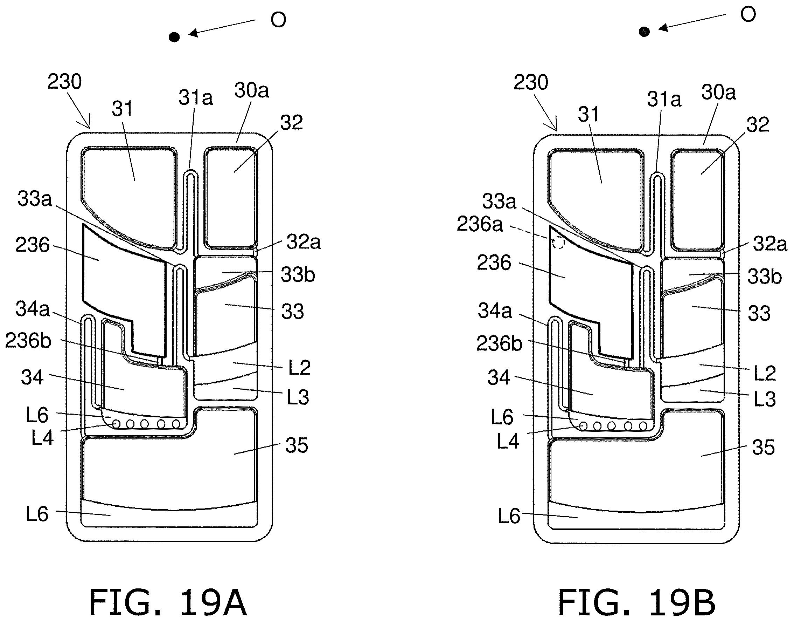

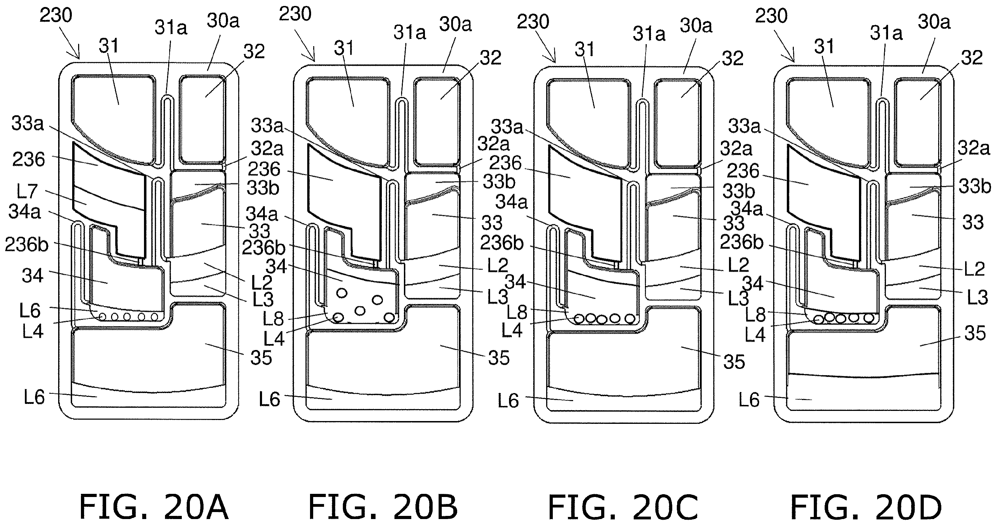

That is, in addition to the configuration discussed above, as shown in Figs. 19A and 19b, the container 230 is equipped with the cleaning liquid chamber 236 disposed inside the collection chamber 34 in the radial direction of a circle centered on the rotation center O, an introduction hole 236a for introducing a cleaning liquid L7 (see Fig. 20A, etc.) into the cleaning liquid chamber 236, and a capillary flow path 236b that connects the cleaning liquid chamber 236 and the collection chamber 34.

The cleaning liquid chamber 236 is positioned between the specimen chamber 31 and the collection chamber 34 in the radial direction of a circle centered on the rotation center O, and the cleaning liquid L7 is manually introduced through the introduction hole 236a provided to a cover that covers the top surface of the container 230 shown in Fig. 19B (see Fig. 20A).

As shown in Fig. 20A, etc., the capillary flow path 236b is provided running along a direction substantially parallel to the radial direction of a circle centered on the rotation center O, and sends the cleaning liquid introduced into the cleaning liquid chamber 236 to the collection chamber 34 by capillary action.

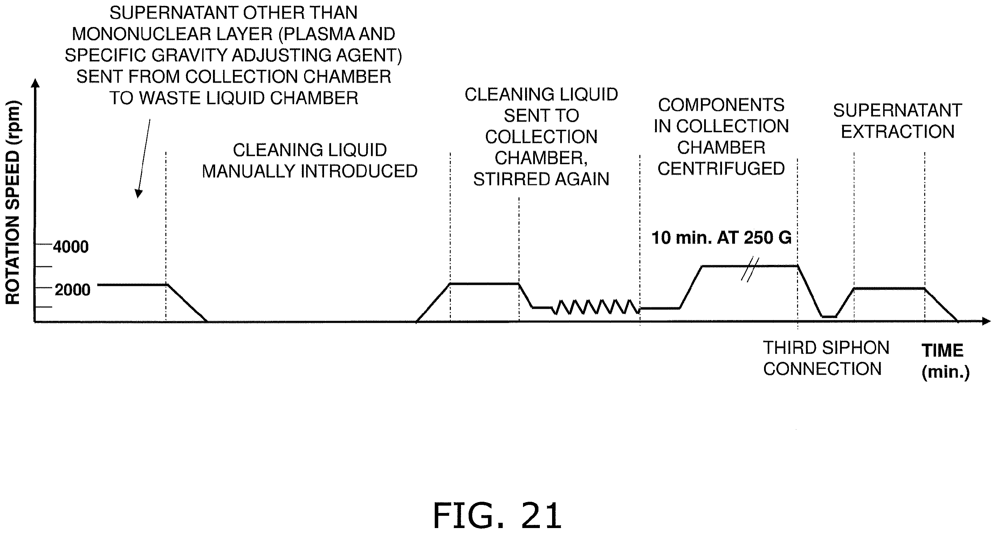

Here, prior to the introduction of the cleaning liquid L7 into the cleaning liquid chamber 236, the container 230 is rotated at approximately 2000 rpm as shown in Fig. 21 in order to send the supernatant liquid other than the mononuclear cell layer L4 (the plasma L5 and some of the specific gravity adjusting agent L2) from the collection chamber 34 to the waste liquid chamber 35 (corresponding to the eighth mode shown in Fig. 12D).

When the cleaning liquid L7 is manually introduced into the cleaning liquid chamber 236, the container 230 is no longer rotating, as shown in Fig. 21.

The cleaning liquid L7 manually introduced into the cleaning liquid chamber 236 is a 0.9% NaCl solution, for example, and is sent via the capillary flow path 236b to the collection chamber 34 in which the suspension L6 containing the mononuclear cell layer L4 is held, as shown in Fig. 20B.

Here, as shown in Fig. 20B, the container 230 is held for a certain length of time in a state in which the rotation speed has been raised to about 2000 rpm as shown in Fig. 21 in order to send the cleaning liquid L7 in the cleaning liquid chamber 236 to the collection chamber 34. After the cleaning liquid L7 has been sent to the collection chamber 34, the rotation speed is lowered to about 1000 rpm to agitate the liquid in the collection chamber 34.

Then, as shown in Fig. 20C, the container 230 is rotated so as to apply a centrifugal force of 250 G for 10 minutes as shown in Fig. 21 in order to centrifuge the mononuclear cell layer L4 contained in the stirred liquid L8 in the collection chamber 34.

Finally, in the state shown in Fig. 20C in which the mononuclear cell layer L4 has been centrifuged, the container 230 is rotated so that the rotation speed, which had been temporarily lowered, is raised again to approximately 2000 rpm as shown in Fig. 21 in order to discharge the supernatant liquid (mixed liquid L8) containing a large amount of platelets as shown in Fig. 20D.

This allows the supernatant portion of the stirred liquid L8 to be discharged to the waste liquid chamber 35 via the siphon flow path 34a.

Consequently, the platelets contained in the collection solution can be removed in the container 230, which simplifies the necessary operations. Furthermore, collecting the mononuclear cell layer L4 after removing the platelets in the container 230 allows mononuclear cell aggregation by the platelets to be suppressed.

In the above description, the configuration is such that after collection of the liquid containing the mononuclear cell layer L4, the rotation of the container 230 is stopped and the cleaning liquid L7 is manually introduced into the cleaning liquid chamber 236 through the introduction hole 236a in the cover, after which the container 230 is rotated to send the cleaning liquid L7 under centrifugal force from the outer periphery of the cleaning liquid chamber 236 to the collection chamber 34 via the capillary flow path 236b connecting to the collection chamber 34, but the present invention is not limited to this.

For instance, the cleaning liquid may be introduced into the container ahead of time, that is, the cleaning liquid may be introduced into the cleaning liquid chamber at the initial stage when the specimen and the specific gravity adjusting agent are introduced.

In this case, it is necessary to control the liquid supply so that the cleaning liquid is discharged from the cleaning liquid chamber to the collection chamber after the liquid containing the mononuclear cell layer has been collected in the collection chamber. Therefore, a multi-stage siphon flow path may be provided, or a valve or the like may be provided in the flow path so that the flow path is open during the cleaning step, and a means for controlling this from the outside may be provided.

### Main Features

As shown in Fig. 5, the container 30 in this embodiment includes the isolation chamber 33, the collection chamber 34, the waste liquid chamber 35, the siphon flow path 33a, and the siphon flow path 34a. The isolation chamber 33 holds the specific gravity adjusting agent L2 and the blood L1 that is layered on the specific gravity adjusting agent L2. The collection chamber 34 holds the mononuclear cell layer L4, some of the specific gravity adjusting agent L2, and the plasma L5 contained in the blood L1, which are sent from the isolation chamber 33 and centrifuged in the isolation chamber 33, and after these have been suspended, only the mononuclear cell layer L4 is precipitated outward in the radial direction in which centrifugal force is applied, in the liquid containing the mononuclear cell layer L4, some of the specific gravity adjusting agent L2, the plasma L5 contained in the blood L1, and other such components. The waste liquid chamber 35 is disposed to the outside in the radial direction of the collection chamber 34 centered on the rotation center O, and holds, as waste liquid, the supernatant liquid sent from the collection chamber 34 in a state in which only the mononuclear cell layer L4 has settled in the collection chamber 34. The siphon flow path 33a connects the isolation chamber 33 and the collection chamber 34, and uses siphoning to send the liquid containing the mononuclear cell layer L4, which has been isolated into components by centrifugal isolation of the blood L1 and the specific gravity adjusting agent L2, from the isolation chamber 33 to the collection chamber 34. The siphon flow path 34a connects the collection chamber 34 and the waste liquid chamber 35, and uses siphoning to send the waste liquid from the collection chamber 34 to the waste liquid chamber 35, from among the liquid held in the collection chamber 34.

Consequently, when a specific component (mononuclear cell layer L4) to be extracted from among the components subjected to the centrifugal isolation is sent to the collection chamber 34, all of the plasma L5 is sent along with some of the specific gravity adjusting agent L2, which reduces the proportion of the specific gravity adjusting agent L2 in the liquid held in the collection chamber 34. Therefore, the adverse effect of the specific gravity adjusting agent L2 on the mononuclear cell layer L4 can be diminished.

As a result, the cells to be detected can be properly isolated and collected without reducing the bioavailability of the cells.

### Embodiment 2

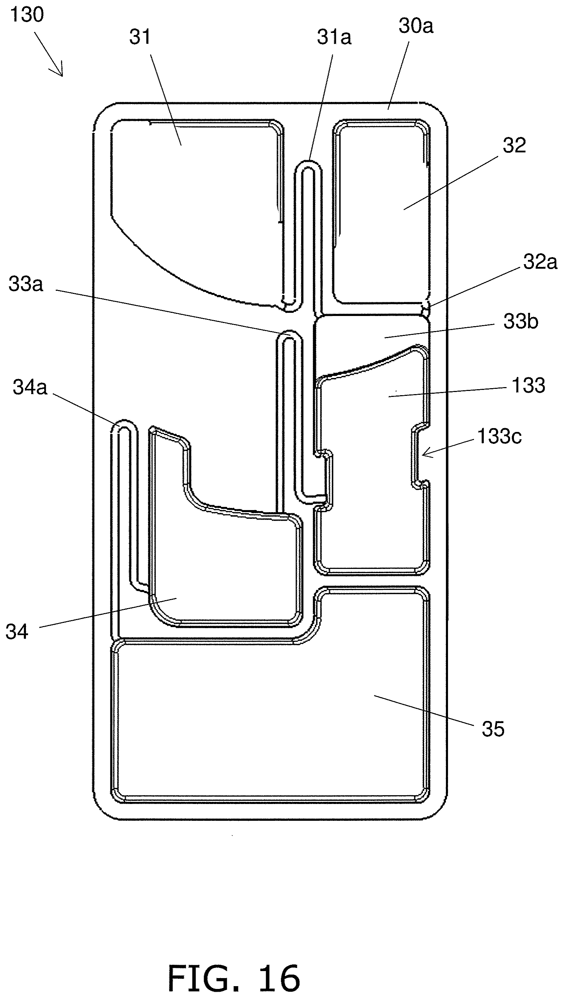

The configuration of the biological sample isolation container (container 130) according to another embodiment of the present invention will now be described with reference to Figs. 16 to 18D.

As shown in Fig. 16, the container 130 of this embodiment differs from Embodiment 1 above in that the main body portion 130a of the container 130 has a constricted portion 133c where the width in a direction intersecting the radial direction is reduced.

The rest of the configuration is the same as that of the container 30 in Embodiment 1, and components that are the same will be numbered the same and described again in detail.

When the blood L1 and the specific gravity adjusting agent L2 sealed in the container 130 are layered and centrifuged, the red blood cells L3, which have a higher specific gravity and are more numerous, move radially outward under the centrifugal force. At this time, since the mononuclear cell layer L4 has a lower specific gravity than the specific gravity adjusting agent L2, it is present as a thin layer between the specific gravity adjusting agent L2 and the layer of plasma L5. After the centrifugation, when the supernatant containing the mononuclear cell layer L4 is isolated through the siphon flow path 33a, there is the risk that the mononuclear cell layer will spill if the liquid interface is disturbed by vibration or the like.

In view of this, with the container 130 in this embodiment, the isolation chamber 133 has a constricted portion 133c where the width in a direction intersecting the radial direction is reduced.

The constricted portion 133c is formed by two protrusions provided on the side wall surfaces of the isolation chamber 133 provided in the radial direction.

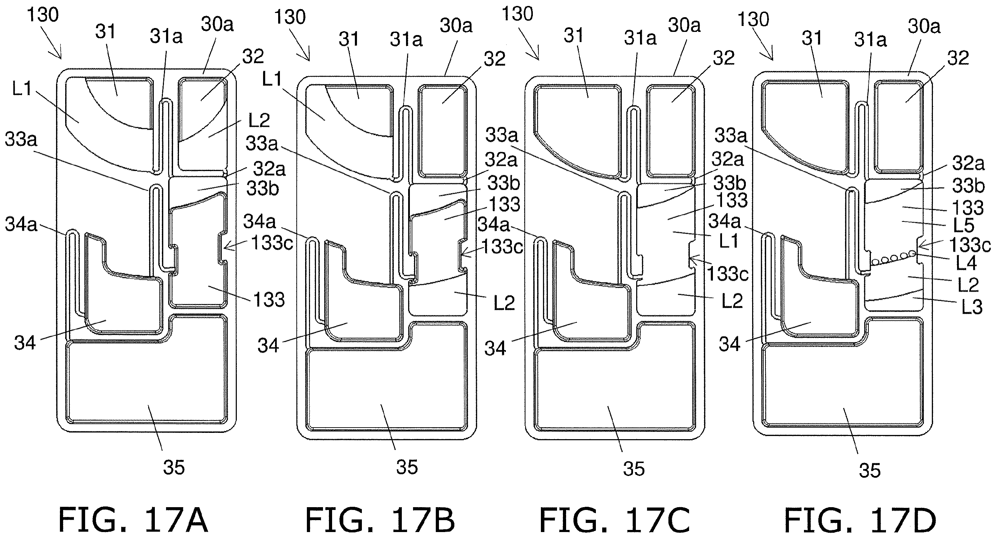

In the step following the transfer of the specific gravity adjusting agent L2 from the reagent chamber 32 to the isolation chamber 133 as shown in Figs. 17A and 17B, and the transfer of the blood L1 from the specimen chamber 31 to the isolation chamber 133, the constricted portion 133c is disposed more to the inside in the radial direction than the interface between the specific gravity adjusting agent L2 and the blood L1 in a state in which the blood L1 has been layered on the specific gravity adjusting agent L2 while centrifugal force is applied within the isolation chamber 133, as shown in Fig. 17C.

Also, as shown in Fig. 17D, the constricted portion 133c is provided at a position corresponding to the radial isolation position where the mononuclear cell layer L4 contained in the liquid obtained by centrifuging the specific gravity adjusting agent L2 and the blood L1 is located.

This allows the centrifuged mononuclear cell layer L4, the plasma L5 having a lower specific gravity than the mononuclear cell layer L4, and some of the specific gravity adjusting agent L2 to be sent to the collection chamber 34 via the siphon flow path 33a.

As discussed above, the container 130 of this embodiment is provided with the constricted portion 133c where the width intersecting the radial direction in which the centrifugal force is applied to the isolation chamber 133 is reduced.

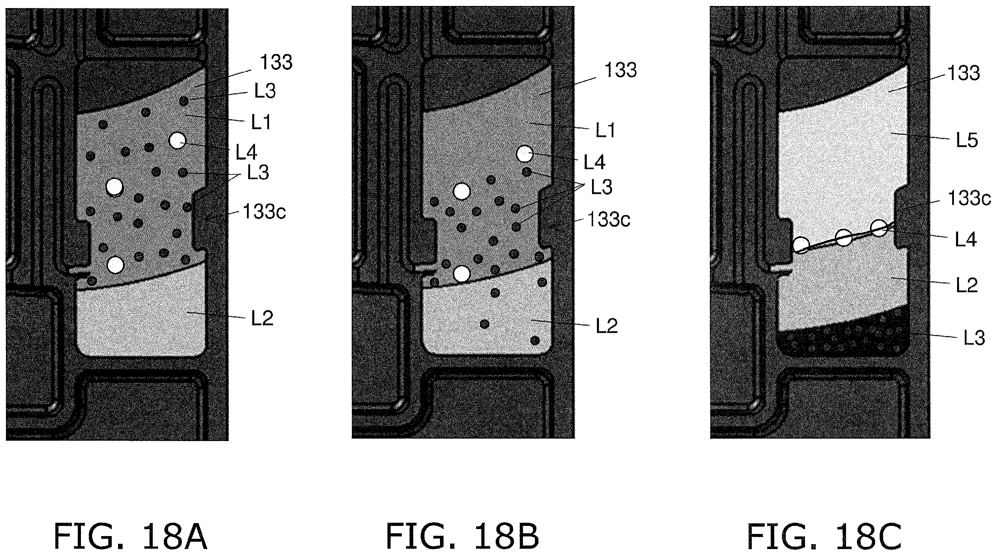

As a result, as shown in Figs. 18A and 18B, when the blood L1 is layered on the upper layer of the specific gravity adjusting agent L2 in the isolation chamber 133, the liquid level of the specific gravity adjusting agent L2 is located below (to the outside in the radial direction of) the constricted portion 133c, and after centrifugation, the interface between the specific gravity adjusting agent L2 and the plasma L5 is located at the constricted portion 133c, as shown in Fig. 18C.

This makes it less likely that the blood cell density will increase at the interface between the blood L1 and the specific gravity adjusting agent L2 located radially outside the constricted portion 133c, as shown in Fig. 18B, even when there is a large amount of red blood cells L3 in the blood L1.

This makes it less likely that the mononuclear cell layer L4 will settle along with the red blood cells L3, which tends to occur when the blood cell density at the interface between the blood L1 and the specific gravity adjusting agent L2 is high, and also prevents the mononuclear cell layer L4 from being disturbed during centrifugation, thereby reducing spillage of the mononuclear cell layer L4.

Here, the shape of the constricted portion 133c provided to the isolation chamber 133 needs to be optimized to suit the shape of the isolation chamber 133 and the rotation speed of the motor 16.

In this embodiment, the isolation chamber 133 is connected to a siphon flow path 33a formed by a capillary tube having a width of 0.6 mm and a depth of 0.2 mm, and is located at a position with a radius of 35 mm from the rotational center, with the overall dimensions being 9.8 mm in width, 17.8 mm in length, and 14 mm in depth, for example.

Because the isolation chamber 133 is provided with the constricted portion 133c where the width in the circumferential direction is reduced, the siphon flow path 33a is connected to the constricted portion 133c, and the width is reduced to 7.8 mm in a positional range of 31 to 36 mm in the radial direction.

The constricted portion 133c is provided so that the ratio of the width of the isolation chamber 133 to the width of the portion other than the constricted portion 133c is 0.5 to 0.8.

This makes it unlikely that the mononuclear cell layer L4 will be engulfed by the red blood cells L3 and moved radially outward, and also makes it unlikely that the mononuclear cell layer L4 will be disturbed during the centrifugation.

### Other Embodiments

An embodiment of the present invention was above, but the present invention is not limited to or by the above embodiment, and various modifications are possible without departing from the gist of the invention.
(A)
   In the above embodiment, an example was given in which the present invention was realized as a biological sample isolation method in which the control unit 20 of the biological sample isolation device 10 in which the containers 30 were placed controlled the rotation of the biological sample isolation device 10. However, the present invention is not limited to this.

For example, the present invention may be realized as a biological sample isolation program that causes a computer to execute the above-mentioned biological sample isolation method.

This biological sample isolation program is stored in a memory (storage unit) installed in a biological sample isolation device, and the CPU reads the biological sample isolation program stored in the memory and causes the hardware to execute the steps. More specifically, the CPU reads the biological sample isolation program and executes the above-mentioned first mode and second mode, thereby achieving the same effects as described above.

The present invention may also be realized as a recording medium for storing a biological sample isolation program.

(B)
In Embodiment 1 described above, an example was given in which the stepped portion 33b where the dimension in the depth direction was reduced was provided to a portion of the isolation chamber 33 on the inside in the radial direction. However, the present invention is not limited to this.

For example, the isolation chamber may be configured without a step.

However, as described above, providing a stepped portion can suppress the influence of disturbance at the gas-liquid interface and avoid variance in the mononuclear cell layer L4, and from that standpoint, it is preferable to use a configuration in which a stepped portion is provided as in Embodiment 1 above.

(C)
In Embodiment 2 above, an example was given in which the constricted portion 133c was formed by two protrusions provided on the side wall surfaces on the two sides of the isolation chamber 133 provided along the radial direction. However, the present invention is not limited to this.

For example, the constricted portion may be a portion in which the width is partially reduced by a single protrusion protruding from one side wall surface of the isolation chamber.

(D)
In the above embodiment, an example was given in which centrifugation was performed in a state in which the blood L1 and the specific gravity adjusting agent L2 had been layered, a liquid containing the mononuclear cell layer L4 (some of the specific gravity adjusting agent L2 and the plasma L5) among the components isolated according to specific gravity was sent to the collection chamber 34, and the rotation speed of the motor 16 was changed stepwise when sending the plasma L5 from the collection chamber 34 to the waste liquid chamber 35. However, the present invention is not limited to this.

For example, the rotation speed in each step is not limited to the number of rotations described in the above embodiment, and can be changed as appropriate depending on the specific component to be isolate and collected, the type of biological sample, the type of reagent, the size of the container, and so forth.

(E)
In the above embodiment, an example was given in which the blood L1 was used as the biological sample from which a specific component was extracted by centrifugal isolation, but the present invention is not limited to this.

For example, the biological sample may be some other biological sample besides blood.

(F)
In the above embodiment, an example was given in which the specific gravity adjusting agent L2 was used as the reagent added to the biological sample (blood L1), but the present invention is not limited to this.

For example, the reagent added to the biological sample may be a reagent other than a specific gravity adjusting agent.

(G)
In the above embodiment, an example was given in which the mononuclear cell layer L4 was used as the specific component extracted from the biological sample (blood L1), but the present invention is not limited to this.

For example, the specific component extracted from the biological sample may be a component other than a mononuclear cell layer.

### INDUSTRIAL APPLICABILITY

The biological sample isolation container of the present invention exhibits the effect that cells to be detected can be properly isolated and collected without reducing the bioavailability of the cells, and is therefore broadly applicable to various kinds of container used in the isolation of biological samples.

### REFERENCE SIGNS LIST

- 10: biological sample isolation device (rotating device)
- 11: housing
- 11a, 11b: opening
- 12: tray section
- 12a: container holder
- 13: base member
- 13a: opening
- 14: display unit
- 15: power button
- 16: motor (rotation drive unit)
- 17: substrate unit
- 20: control unit (biological sample isolation control device, rotation control unit)
- 30: container (biological sample isolation container)
- 30a: main body portion
- 31: specimen chamber
- 31a: siphon flow path (third siphon flow path)
- 32: reagent chamber
- 32a: capillary flow path
- 33: isolation chamber
- 33a: siphon flow path (first siphon flow path)
- 33b: stepped portion
- 34: collection chamber
- 34a: siphon flow path (second siphon flow path)
- 35: waste liquid chamber
- 36: lid member
- 36a: introduction hole (first introduction hole)
- 36b: introduction hole (second introduction hole)
- 36c: collection hole
- 36d: air hole
- 130: container (biological sample isolation container)
- 130a: main body portion
- 133: isolation chamber
- 133c: constricted portion
- 236: cleaning liquid chamber
- 236a: introduction hole
- 236b: capillary flow path
- d: distance
- L1: blood (biological sample)
- L2: gravity adjusting agent (reagent)
- L3: red blood cells
- L4: mononuclear cell layer
- L5: plasma (first component)
- L6: suspension (liquid)
- L7: cleaning liquid
- L8: stirred liquid
- O: rotation center
- V1-V4, V6-V10: speeds
- V5: speed range

## Claims

1. A biological sample isolation container that is placed in a rotating device and rotates around a specific rotation center to apply centrifugal force, thereby collecting a specific component contained in a biological sample that has been isolated for each component, the biological sample isolation container comprising:
an isolation chamber configured to hold a reagent and a biological sample layered on the reagent;
a collection chamber configured to hold the specific component, some of the reagent, and a first component contained in the biological sample, which have been sent from the isolation chamber and have been centrifuged in the isolation chamber, and after these have been suspended, cause only the specific component to settle outward in a radial direction in which centrifugal force is applied in a liquid containing the specific component, some of the reagent, and the first component contained in the biological sample;
a waste liquid chamber that is disposed on the outside in the radial direction of the collection chamber around the rotation center, and that is configured to hold as waste liquid a supernatant liquid of the liquid sent from the collection chamber, in a state in which only the specific component has settled in the collection chamber;
a first siphon flow path that connects the isolation chamber and the collection chamber, and is configured to use siphoning to send the liquid, which contains a specific component that has been isolated for each component by centrifuging of the biological sample and reagent, from the isolation chamber to the collection chamber; and
a second siphon flow path that connects the collection chamber and the waste liquid chamber, and is configured to use siphoning to send the waste liquid from the collection chamber to the waste liquid chamber, out of the liquid stored in the collection chamber.

2. The biological sample isolation container according to claim 1, further comprising:
a specimen chamber that is disposed to an inside in the radial direction of the isolation chamber around the rotation center, and is configured to hold the biological sample; and
a third siphon flow path that connects the specimen chamber and the isolation chamber, and is configured to use siphoning to send the biological sample from the specimen chamber to the isolation chamber.

3. The biological sample isolation container according to claim 1 or 2, further comprising:
a reagent chamber that is disposed to an inside in the radial direction of the isolation chamber around the rotation center, and is configured to hold the reagent; and
a capillary flow path that connects the reagent chamber and the isolation chamber, and is configured to use capillary action to send the reagent from the reagent chamber to the isolation chamber.

4. The biological sample isolation container according to claim 1 or 2,
wherein the first siphon flow path and the second siphon flow path are disposed on an upper side in a vertical direction in which gravity acts on the liquid when the liquid is held in the isolation chamber, the collection chamber, and the waste liquid chamber,
the first siphon flow path connects the isolation chamber and the collection chamber on the upper side in the vertical direction, and
the second siphon flow path connects the collection chamber and the waste liquid chamber on the upper side in the vertical direction.

5. The biological sample isolation container according to claim 3,
wherein the capillary flow path is disposed on an upper side in the vertical direction in which gravity acts on the liquid when the liquid is held in the reagent chamber, and
the capillary flow path connects the reagent chamber and the isolation chamber on the upper side in the vertical direction.

6. The biological sample isolation container according to claim 2,
wherein the third siphon flow path is disposed on an upper side in the vertical direction in which gravity acts on the liquid when the liquid is held in the specimen chamber and the isolation chamber, and
the third siphon flow path connects the specimen chamber and the isolation chamber on the upper side in the vertical direction.

7. The biological sample isolation container according to claim 1 or 2,
further comprising a main body portion on an upper surface of which are provided the isolation chamber, the collection chamber, the waste liquid chamber, the first siphon flow path, and the second siphon flow path, and a lid member that is attached so as to cover the main body portion,
wherein the first siphon flow path and the second siphon flow path are formed between a recess formed on the upper surface of the main body portion and the lid member.

8. The biological sample isolation container according to claim 7, further comprising:
a specimen chamber that is disposed to an inside in the radial direction of the isolation chamber around the rotation center, and is configured to hold the biological sample; and
a third siphon flow path that connects the specimen chamber and the isolation chamber, and is configured to use siphoning to send the biological sample from the specimen chamber to the isolation chamber,
wherein the third siphon flow path that connects the specimen chamber and the isolation chamber and uses siphoning to send the biological sample from the specimen chamber to the isolation chamber is formed between a recess formed on the upper surface of the main body portion and the lid member.

9. The biological sample isolation container according to claim 7, further comprising:
a reagent chamber that is disposed to the inside in the radial direction of the isolation chamber around the rotation center, and is configured to hold the reagent; and
a capillary flow path that connects the reagent chamber and the isolation chamber, and is configured to use capillary action to send the reagent from the reagent chamber to the isolation chamber,
wherein the capillary flow path that connects the reagent chamber and the isolation chamber and uses capillary action to send the reagent from the reagent chamber to the isolation chamber is formed between a recess formed on the upper surface of the main body portion and the lid member.

10. The biological sample isolation container according to claim 7,
wherein the lid member has a first introduction hole is configured to introduce the biological sample, a second introduction hole is configured to introduce the reagent, a collection hole is configured to collect the specific component, and a plurality of air holes.

11. The biological sample isolation container according to claim 1 or 2,
wherein the first siphon flow path is connected at its first end to a surface of a side wall constituting the isolation chamber that runs along the radial direction centered on the rotation center, and at its second end on an opposite side from the first end to a surface on an inside diameter side of the side wall constituting the collection chamber that intersects with the radial direction.

12. The biological sample isolation container according to claim 11,
wherein the first siphon flow path is connected at its first end to a portion of the side wall surface where the specific component subjected to the centrifugal isolation is located, at a position on the outside in the radial direction.

13. The biological sample isolation container according to claim 1 or 2,
wherein the second siphon flow path is connected at its first end to a surface of the side wall constituting the collection chamber that runs along the radial direction centered on the rotation center, and at its second end on an opposite side from the first end to the surface on an inside diameter side of the side wall constituting the waste liquid chamber that intersects with the radial direction.

14. The biological sample isolation container according to claim 2,
wherein a first end of the third siphon flow path is connected to a portion of the side wall constituting the specimen chamber that is located near the intersection between a surface that runs along the radial direction around the rotation center and a surface intersecting with the radial direction, and a second end that is on an opposite side from the first end is connected to a surface of the side wall constituting the isolation chamber on an inside diameter side intersecting with the radial direction.

15. The biological sample isolation container according to claim 3,
wherein a first end of the capillary flow path is connected to an outer surface of the side wall constituting the reagent chamber in the radial direction centered on the rotation center, and a second end on the opposite side from the first end is connected to a surface of the side wall constituting the isolation chamber on an inside diameter side intersecting with the radial direction.

16. The biological sample isolation container according to claim 1 or 2,
wherein the isolation chamber further has a stepped portion at a position to an inside in the radial direction, the stepped portion having a height in a vertical direction that is less than that at a position to an outside in the radial direction.

17. The biological sample isolation container according to claim 16,
wherein the stepped portion is provided at a portion where a first flow path configured to send the biological sample to the isolation chamber and a second flow path configured to send the reagent are connected.

18. The biological sample isolation container according to claim 16,
wherein the stepped portion is provided at a filling position in the isolation chamber of the liquid obtained by centrifugal isolation of the biological sample and the reagent under a state in which centrifugal force is applied.

19. The biological sample isolation container according to claim 16,
wherein the depth of the stepped portion the vertical direction is less than a distance between an isolation position in the radial direction at which the specific component contained in a liquid obtained by centrifuging the reagent and the biological sample is located, and the inner wall surface in the radial direction of the isolation chamber.

20. The biological sample isolation container according to claim 1 or 2,
wherein the isolation chamber further has a constricted portion in the radial direction, where the dimension in a width direction substantially perpendicular to the radial direction is less than that of other portions.

21. The biological sample isolation container according to claim 20,
wherein the constricted portion is disposed to an inside in the radial direction of the interface between the reagent and the biological sample in a state in which the biological sample is layered on the reagent while centrifugal force is being applied in the isolation chamber.

22. The biological sample isolation container according to claim 20,
wherein the constricted portion is provided at a position corresponding to an isolation position in the radial direction at which the specific component contained in the liquid obtained by centrifuging the reagent and the biological sample is located.

23. The biological sample isolation container according to claim 20,
wherein the constricted portion is a convex portion provided to both side wall surfaces of the isolation chamber, running along the radial direction.

24. The biological sample isolation container according to claim 20,
wherein the first siphon flow path is connected to the isolation chamber at the constricted portion.

25. The biological sample isolation container according to claim 20,
wherein the constricted portion is provided so that the ratio of the width of the constricted portion to the width of the portion of the isolation chamber other than the constricted portion is 0.5 to 0.8.

26. A biological sample isolation control device that controls rotation of a rotating device in which the biological sample isolation container according to claim 1 or 2 is placed, the biological sample isolation control device comprising:
a rotation control unit is configured to control a drive unit of the rotating device.

27. The biological sample isolation control device according to claim 26,
wherein the rotation control unit has:
a first mode in which the drive unit of the rotating device is rotated at a first speed so that the biological sample and the reagent are layered in the isolation chamber in a state of having been brought closer to the outer surface in the radial direction; and
a second mode in which the drive unit is rotated at a second speed that is higher than the first speed to centrifuge the biological sample and the reagent.

28. The biological sample isolation control device according to claim 27,
wherein the rotation control unit further has:
a third mode in which, after the biological sample and the reagent are centrifuged in the second mode, the drive unit is rotated at a third speed that is lower than the first speed and the second speed, and a liquid containing the specific component is allowed to flow into the first siphon flow path.

29. The biological sample isolation control device according to claim 28,
wherein the rotation control unit further has:
a fourth mode in which, after the third mode, the drive unit is rotated at a fourth speed that is higher than the third speed, and the liquid containing the specific component is sent from the isolation chamber to the collection chamber.

30. The biological sample isolation control device according to claim 29,
wherein the rotation control unit further has:
a fifth mode further in which, after the fourth mode, the rotation speed is raised or lowered in a speed range that is lower than the fourth speed so as to suspend the liquid containing the specific component sent to the collection chamber.

31. The biological sample isolation control device according to claim 30,
wherein the rotation control unit further has:
a sixth mode in which, after the fifth mode, the drive unit is rotated at a sixth speed that is higher than the speed range to centrifuge the liquid containing the specific component, thereby causing the specific component to settle on the outer surface in the radial direction.

32. The biological sample isolation control device according to claim 31,
wherein the rotation control unit further has:
a seventh mode in which, after the sixth mode, the drive unit is rotated at a seventh speed that is lower than the sixth speed to cause the first component excluding the specific component to flow into the second siphon flow path.

33. The biological sample isolation control device according to claim 32,
wherein the rotation control unit further has:
an eighth mode in which, after the seventh mode, the drive unit is rotated at an eighth speed that is higher than the seventh speed, and the first component is sent from the collection chamber to the waste liquid chamber.

34. The biological sample isolation control device according to claim 27,
wherein the biological sample isolation container further comprises:
a reagent chamber that is disposed to the inside in the radial direction of the isolation chamber around the rotation center, and is configured to hold the reagent; and
a capillary flow path that connects the reagent chamber and the isolation chamber, and is configured to use capillary action to send the reagent from the reagent chamber to the isolation chamber, and
the rotation control unit further has:
a ninth mode in which the drive unit is rotated at a ninth speed that is lower than the first speed, and the reagent is sent from the reagent chamber to the isolation chamber through the capillary flow path.

35. The biological sample isolation control device according to claim 34,
wherein the biological sample isolation container further comprises:
a specimen chamber that is disposed to the inside in the radial direction of the isolation chamber around the rotation center, and is configured to hold the biological sample;
and
a third siphon flow path that connects the specimen chamber and the isolation chamber, and is configured to use siphoning to send the biological sample from the specimen chamber to the isolation chamber, and
the rotation control unit further has:
a tenth mode in which the drive unit is rotated at a tenth speed that is lower than the ninth speed to cause the biological sample to flow into the third siphon flow path.

36. The biological sample isolation control device according to claim 35,
wherein the rotation control unit further has:
an eleventh mode in which the drive unit is rotated at the first speed, which is higher than the tenth speed, and the biological sample is sent through the third siphon flow path to the isolation chamber so that the biological sample is layered on the reagent.

37. A biological sample isolation control method, featuring a biological sample isolation control device that controls the rotation of a rotating device in which the biological sample isolation container according to claim 1 or 2 is placed, the biological sample isolation control method comprising:
a first step of rotating a drive unit of the rotating device at a first speed so that the biological sample and the reagent are layered in a state of having been brought close to the outer surface in the radial direction in the isolation chamber; and
a second step of rotating the drive unit at a second rotation speed that is higher than the first speed to centrifuge the biological sample and the reagent.

38. A biological sample isolation control program,
which causes a computer to execute the biological sample isolation control method according to claim 37.
